# EUROPEAN PATENT APPLICATION

(11) **EP 4 446 743 A1**
(43) Date of publication of application: **16.10.2024**
(21) Application number: 23382347.5
(22) Date of filing: 14.04.2023
(51) Int. Cl.: G01N 33/58, A61K 49/08, A61K 49/14, G01N 33/94

(54) **PROTEIN-BASED BIOSENSOR SUITABLE FOR MRI IMAGING**

(71) Applicant: Fundación Instituto de Investigación Sanitaria de Santiago de Compostela, 15706 Santiago de Compostela (ES)
(72) Inventor: HERVELLA LORENZO, Pablo, 15706 Santiago de Compostela (ES); IGLESIAS REY, Ramón, 15706 Santiago de Compostela (ES); CASTILLO SÁNCHEZ, Jose, 15706 Santiago de Compostela (ES)
(74) Representative: Hoffmann Eitle

(57) **Abstract**

The present invention refers to the medical field. Particularly, the present invention refers to a protein-based biosensor comprising a substrate-binding protein bound to an MRI contrast agent by means of a chelating agent.

## Description

### FIELD OF THE INVENTION

The present invention refers to the medical field. Particularly, the present invention refers to a protein-based biosensor comprising a substrate-binding protein bound to an MRI contrast agent by means of a chelating agent. The invention can be implemented to detect substrates that are not easily accessible through optical imaging techniques. For instance, it could be used for the quantification of neurotransmitters in the brain, thereby promoting the development of diagnostic methods for neurological disorders.

### STATE OF THE ART

Neurotransmitters are molecules that amplify, transmit, and convert signals in cells, having an essential role in information transmission throughout the nervous system. Hundreds of such chemicals have been discovered in the last century, continuing to be identified and studied concerning their action on brain health. These substances have been observed to influence numerous functions, including emotions, thoughts, memories, learning, and movements. Thus, disturbances in neurotransmitters' homeostasis started being correlated with a plethora of neurological and neurodegenerative disorders, including epilepsy, multiple sclerosis, amyotrophic lateral sclerosis, Alzheimer's disease, Parkinson's disease, Huntington's disease, depression and, importantly, stroke.

Stroke is the second cause of death worldwide and the first cause of disability. Improvements in the management of stroke patients, especially in developed countries, have contributed to the reduction in mortality and morbidity rates observed in recent decades. However, the incidence of stroke did not follow the same trend with a continuous increase in developed countries. Recent statistics released by the world stroke organization showed a 70% increase in incident strokes and a 43% of deaths from stroke in the last 20 years, with an estimated global cost over US$721 billion. In this context, the identification of targets that allow an early diagnosis and a preventive treatment in high-risk subjects is an essential objective.

The endothelium has a pivotal role in cerebrovascular disease. The blood-brain barrier (BBB) is a highly selective semipermeable border of endothelial cells that prevents solutes in the circulating blood from non-selectively crossing into the extracellular fluid of the central nervous system where neurons reside. It represents a biological and mechanical barrier between the cerebral and vascular compartments.

The BBB endothelium is indispensable for regulating tonicity and vascular homeostasis. In healthy vascular endothelium, these functions are regulated through several mechanisms, such as vasodilatation, antioxidation, anti-inflammation, inhibition of adhesion and leukocyte migration, inhibition of smooth muscular fibers, inhibition of platelets adhesion and aggregation, and anticoagulant and profibrinolytic effects. An alteration in these mechanisms is generally reflective of the presence of endothelial disfunction. For instance, atherosclerosis - which is associated to endothelial dysfunction - can be readily diagnosed by ultrasound or through several biomarkers of oxidative stress, adhesion molecule isoforms, inflammation, endothelial progenitor cells, etc. However, these markers of endothelial dysfunction are not associated neither with the development nor the severity of stroke.

Recently it has been possible to demonstrate other biological effects of the BBB related to glutamate transport. Glutamate excitotoxicity refers to the process by which excessive activation of glutamate receptors leads to neuronal damage and death. Glutamate is the main excitatory neurotransmitter in the brain and plays an important role in many physiological processes. However, when levels of glutamate become too high, it can cause overstimulation of neurons, leading to the production of reactive oxygen species (ROS) and calcium overload, which ultimately result in cell death. Glutamate excitotoxicity is thought to play a role in multiple neurological disorders, including stroke, multiple sclerosis, traumatic brain injury, and neurodegenerative diseases such as Alzheimer's and Parkinson's disease. In these conditions, excess glutamate release causes a surge of calcium ions into neurons, triggering a cascade of events that can ultimately lead to neuronal death.

The concentration of extracellular glutamate is tightly regulated to maintain physiological concentrations through sodium-dependent transporters (also known as excitatory amino acid transporters: EAATs). Reuptake of glutamate from synaptic junctions after neuron excitation normally involves the participation of EAATs located on astrocytes, which bind and remove the neurotransmitter for processing and recycling. Alternatively, when extracellular concentrations become elevated, sodium-dependent transporters located on the antiluminal surface of brain capillaries introduce glutamate from the extracellular space into endothelial cells. When glutamate accumulates in endothelial cells to a concentration that exceeds plasma levels it is moved via facilitated diffusion through the luminal side into the blood stream into the circulation. In this regard, endothelial regulation of glutamate in the central nervous system (CNS) can occur despite unfavorable concentration gradients from CNS to plasma.

Based on the efficient capacities of astrocytes and endothelial cells to remove and metabolize glutamate, and because they are less susceptible than neurons to ischemic conditions, it could be postulated that both cellular components of the neurovascular unit should be able to protect the neurons against the glutamate excitotoxicity. However, experimental and clinical evidence show that those endogenous homeostatic mechanisms are not able to stop the glutamate effect after ischemic stroke.

Clinical studies demonstrated that glutamate is critical for neuronal damage after ischemic stroke. In that regard, ischemic stroke patients presented higher blood and cerebrospinal fluid (CSF) glutamate levels than control subjects at admission, suggesting that glutamate concentrations above 200 µM in plasma acted as an important predictor of neuronal damage progression at 48 h, with a sensitivity of 85% and a specificity of 97%. Moreover, high levels of glutamate in plasma for at least 24 h were associated with early neurological deterioration, whereas in patients with stable stroke, glutamate levels dropped to normal values less than 6 h from onset. All these clinical data demonstrated for the first time the critical role of glutamate in stroke pathology and suggest a source of new strategies for the research of new protective therapies based on the inhibition of glutamate toxicity. Therefore, controlling the increase of extracellular glutamate may confer neuroprotection by terminating multiple downstream death signalling cascades at their converging upstream initiation point. More recently, it was demonstrated that glutamate scavengers capable of decreasing the glutamate concentrations in blood after i.v. injection efficiently reduced the excitotoxicity of glutamate in ischemic stroke models, showing the potential neuroprotective effects of the glutamate reducing drugs.

Reducing extracellular excess of glutamate in the brain is a key target to eliminate excitotoxity and reducing neuronal death and neurodegeneration in many neurological diseases.

Due to the importance of glutamate, and many other neurotransmitters' homeostasis in neurological and neurodegenerative disorders, substantial effort has been put into developing neurotransmitter sensors that could be used to track and quantify neurotransmitters *in vivo* and *in vitro.* In particular, glutamate sensing has been the scope of multiple fundamental studies in biology. Several protein-based glutamate sensors have been developed and tested to measure glutamate concentration at the cellular surface.

Fluorescent sensors are molecules that bright in contact with a specific analyte. In the last decade, a new family of fluorescent sensors have been developed based on fluorescent proteins. These proteins fold in the presence of their targeted analyte, resulting in a new tertiary structure that provides a fluorescent signal (usually through a green fluorescent protein). In this regard, glutamate sensing has been the focus of interest of many research groups, developing several glutamate sensor proteins such as iGluSnFr or iGlu1-3. The main application of these sensors was the study of neuronal activity and synapsis using intravital fluorescence imaging. Although the outcomes of these studies are of utmost importance in the field of neurological diseases, the translation of fluorescent sensor to clinical application is hindered by the limitations of fluorescent imaging, that is unable to visualize deep tissues or obtain images through the bones.

Magnetic Resonance Imaging (MRI) is a non-invasive imaging technique widely used in clinic to study organs, tissues, and bones. Compared to the other imaging techniques used in clinic, positron emission tomography (PET) and X-ray based computerized tomography (CT). MRI provides a higher special resolution, being the preferred technique to diagnose neurological diseases. The main drawback of MRI compared with PET is the lower sensitivity, and due to the use of radioactive tracers providing signal at very low concentrations (at pM levels) PET is usually preferred to study body's functions at cellular levels, such as cellular metabolism or oxygen consumption. This drawback could be partially solved by using contrast agents.

Contrast agents for MRI are currently used to increase the signal to noise ratio, providing images with very high special resolution (around 1 µm), not achievable with other imaging techniques. Among the MRI contrast agents, Gadolinium (Gd) containing contrast agents are the most used in clinic, with 9 types already in the market, being the Gadopiclenol the last Gd-containing contrast agent approved by the FDA in 2022. In 2017 the FDA raised an important concern about the use of Gd and its accumulation in the brain when using Gd physically linked to linear chelators to create the contrast agent. Nowadays, it has been demonstrated that cyclic chelators stably retain Gd and it has been demonstrated the safety of this kind of contrast agents. The main benefits of the already marketed Gd containing contrast agents is the enhancement of the images in the voxels where the Gd is present, resulting in brighter images. This effect has many applications in clinic, being the most used the detection of local lesions (tumors, abscess, metastasis, infarct); imaging of vessels (using angiography or venography); and to calculate perfusion parameters. Other kind of MRI contrast agents are based on nanoparticles, that also provides an MRI signal and are being currently used in several clinical trials with 4 of them already in the market. The main application of both Gd-containing contrast agents and nanoparticles is the visualization of a local dysfunction in the body and is based on the abnormal accumulation of those contrast agents in the region of interest. However, recent studies in the field have demonstrated the capacity of MRI to quantitatively measure concentrations of different molecules, providing a useful analytical tool that could be used also for diagnosis.

Since there is an unmet medical need to develop clinically applicable strategies for the quantification of neurotransmitters that could promote the development of diagnostic methods for neurological disorders, the present invention is focused on solving this problem and a protein-based biosensor suitable for MRI is herein provided, using glutamate as a proof-of-concept substrate.

### DESCRIPTION OF THE INVENTION

### Brief description of the invention

The present invention refers to a protein-based biosensor comprising a substrate-binding protein bound to an MRI contrast agent by means of a chelating agent.

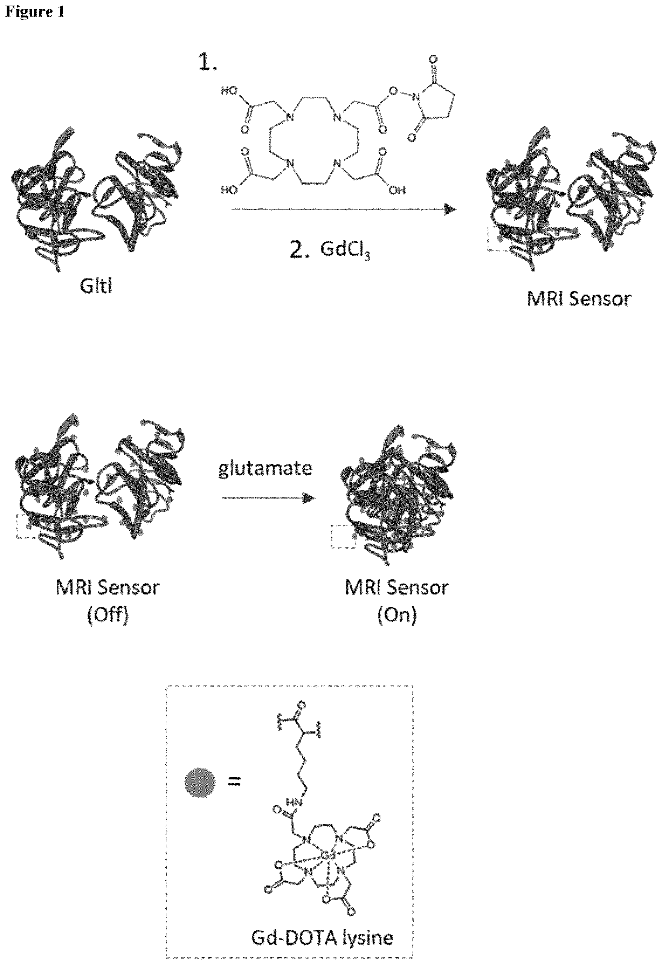

The inventors of the present invention identified the need to develop clinically applicable strategies to detect substrates in tissues that are not easily accessible, and in particular, to quantify neurotransmitters in the brain. They hypothesised that this problem could be solved by generating protein-based biosensors comprising a substrate-binding protein bound to an MRI contrast agent by means of a chelating agent that, upon contact with the substrate, would experience a conformational change that would shift the MRI signal provided by the contrast agent attached to the structure (**Figure 1****).**

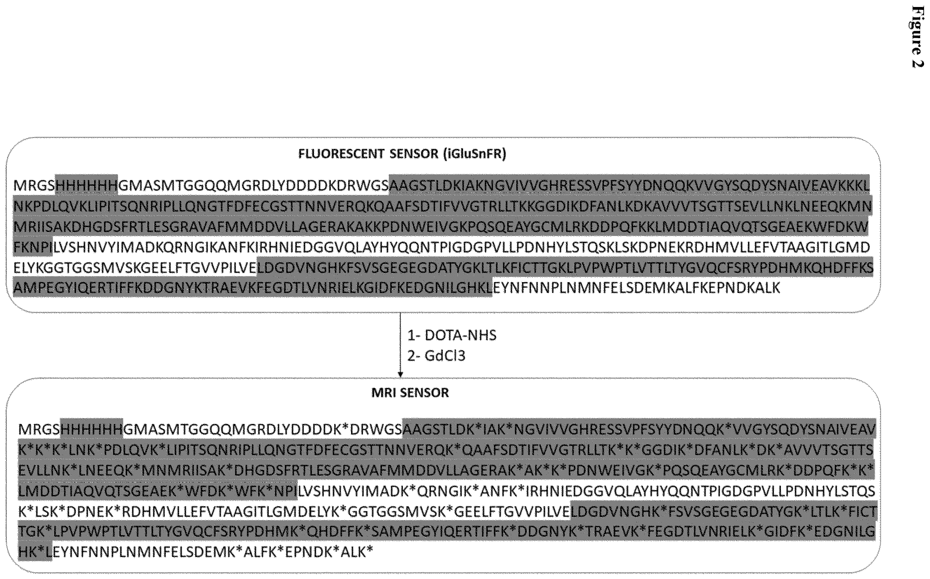

Using glutamate as a proof-of-concept substrate, the inventors generated an MRI glutamate sensor by labelling a glutamate transporter protein (Gltl) with Gd, using DOTA as a chelating agent, by stable amide bonds between lysine residues and DOTA **(****Figure 2****).** This sensor will be referred to as the MRI glutamate sensor. The fluorescent glutamate sensor iGluSnFR was used as a starting point, since apart from containing a Gltl domain, it also has a fluorescent protein reporter (GFP) that was useful for preliminary tests. They also generated two additional sensors using the same synthetic strategy:
- GOT-DOTA-Gd: A glutamate sensor wherein the substrate-binding protein was glutamic oxalacetate transaminase (GOT), an enzyme involved in glutamate metabolism.
- BSA-DOTA-Gd: A bovine serum albumin (BSA) sensor with no specific binding domain for glutamate and with a BSA specific binding domain.

The protein sequences used for the generation of the three biosensors are summarised in **Table 1.**

**Table 1: Protein sequences used for the generation of the biosensors.**

| Biosensor | SEQ ID NO | Protein sequence (N-terminus to C-terminus) |
|---|---|---|
| MRI glutamate sensor | 1 | DRWGSAAGSTLDKIAKNGVIVVGHRESSVPF SYYDNQQKVVGYSQDYSNAIVEAVKKKLNK PDLQVKLIPITSQNRIPLLQNGTFDFECGSTTN NVERQKQAAFSDTIFVVGTRLLTKKGGDIKD FANLKDKAVVVTSGTTSEVLLNKLNEEQKM NMRIISAKDHGDSFRTLESGRAVAFMMDDVL LAGERAKAKKPDNWEIVGKPQSQEAYGCML RKDDPQFKKLMDDTIAQVQTSGEAEKWFDK WFKNPI |
| GOT-DOTA-Gd | 2 | MWLTEAQKFG VAFTFGGFLF FLFGIFTFFD RALLALGNIL FLIGVFLIIG SQKTYIFFTR PNKRRGSLFF LVGAFLILLK WTFLGFIIES LGIIGLFGDF FGVIVQFLRS MPIIGPILSH PAIAPIVDKL AGVRVLPV |
| BSA-DOTA-Gd | 3 | MKWVTFISLLLLFSSAYSRGVFRRDTHKSEIA HRFKDLGEEHFKGLVLIAFSQYLQQCPFDEH VKLVNELTEFAKTCVADESHAGCEKSLHTLF GDELCKVASLRETYGDMADCCEKQEPERNE CFLSHKDDSPDLPKLKPDPNTLCDEFKADEK KFWGKYLYEIARRHPYFYAPELLYYANKYN GVFQECCQAEDKGACLLPKIETMREKVLASS ARQRLRCASIQKFGERALKAWSVARLSQKFP KAEFVEVTKLVTDLTKVHKECCHGDLLECA DDRADLAKYICDNQDTISSKLKECCDKPLLE KSHCIAEVEKDAIPENLPPLTADFAEDKDVCK NYQEAKDAFLGSFLYEYSRRHPEYAVSVLLR LAKEYEATLEECCAKDDPHACYSTVFDKLKH LVDEPQNLIKQNCDQFEKLGEYGFQNALIVR |
| | | YTRKVPQVSTPTLVEVSRSLGKVGTRCCTKP ESERMPCTEDYLSLILNRLCVLHEKTPVSEKV TKCCTESLVNRRPCFSALTPDETYVPKAFDEK LFTFHADICTLPDTEKQIKKQTALVELLKHKP KATEEQLKTVMENFVAFVDKCCAADDKEAC FAVEGPKLVVSTQTALA |

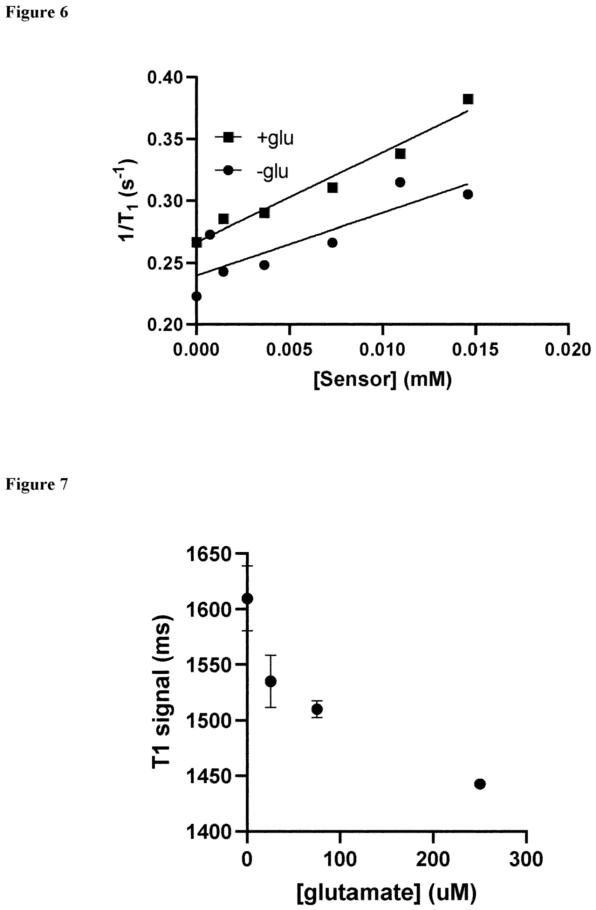

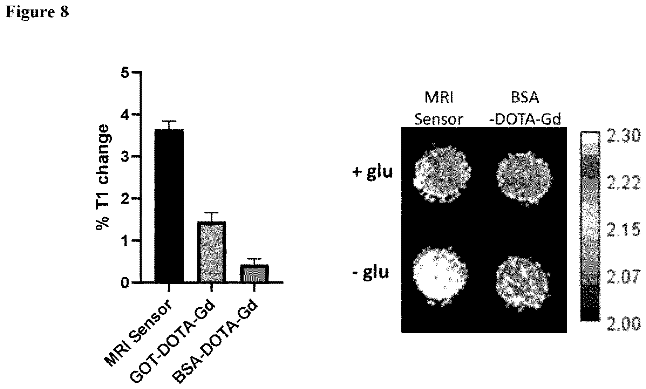

To evaluate the sensing properties of the sensors, the inventors measured the MRI signal generated by the sensor in the presence and absence of substrate. As expected, glutamate caused a shift in the MRI signal generated by the MRI glutamate sensor **(****Figure 6** and **Figure 8****).** More precisely, it reduced the MRI signal in a concentration-dependent manner **(****Figure 7****).** These results evidence that currently available biosensors could be repurposed for MRI.

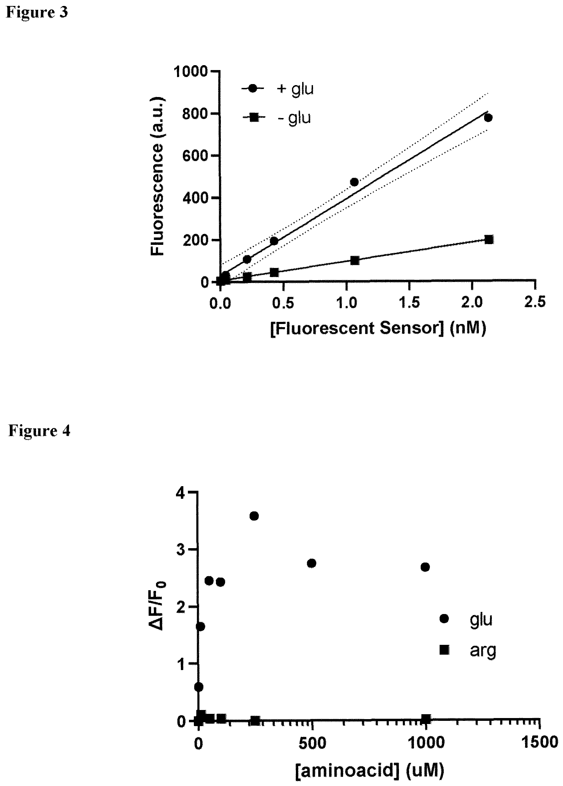

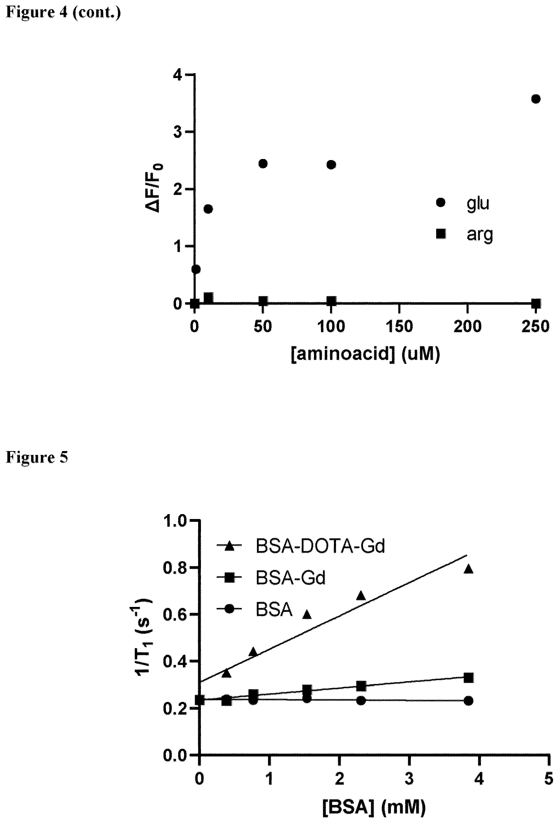

Similarly, glutamate also caused a shift in the MRI signal generated by the GOT-DOTA-Gd sensor, although the effect was lower to that observed for the MRI glutamate sensor **(****Figure 8****).** Importantly, BSA also caused a shift in the MRI signal generated by the BSA-DOTA-Gd sensor **(****Figure 4****),** supporting the applicability the synthesis strategy to other substrates.

These results support the applicability of the synthesis strategy to different types of substrate-binding proteins; particularly suggesting that substrate-specific transporters may be more suitable for the generation of synthetic biosensors than other substrate-binding proteins. The lower signal response of the GOT compared to the sensor could be due to a higher conformational change of the latter in the presence of glutamate.

Consequently, the **first embodiment** of the present invention refers to a protein-based biosensor comprising a substrate-binding protein bound to an MRI contrast agent by means of a chelating agent.

In a preferred embodiment, the protein-based biosensor comprises a neurotransmitter binding protein bound to an MRI contrast agent by means of a chelating agent.

In a preferred embodiment, the protein-based biosensor comprises a glutamate binding protein bound to an MRI contrast agent by means of a chelating agent.

In a preferred embodiment, the substrate-binding protein is bound to an MRI contrast agent by means of a cyclic chelating agent.

In a preferred embodiment, the chelating agent is dodecane tetraacetic acid (DOTA).

In a preferred embodiment, the MRI contrast agent is a gadolinium-based MRI contrast agent.

In a preferred embodiment, the protein-based biosensor comprises a glutamate binding protein bound to gadoteric acid (DOTA-Gd) that consists of dodecane tetraacetic acid (DOTA) as a chelating agent and gadolinium.

In a preferred embodiment, the protein-based biosensor is characterized in that the dodecane tetraacetic acid (DOTA) is bound to the glutamate binding protein by means of amide bonds with lysine amino acids.

In a preferred embodiment, the glutamate binding protein is characterized by SEQ ID NO: 1.

The **second embodiment** of the invention refers to a nanocarrier comprising any of the protein-based sensors described above, characterized in that it is functionalized with a ligand capable to bind to the blood-brain barrier.

In a preferred embodiment, the nanocarrier is characterized in that the ligand is selected from the list comprising: an antibody, a peptide, an aptamer or a small molecule with affinity for at least one receptor at the blood-brain barrier selected from the list comprising: VCAM-1, E-Selectin, PECAM-1, CD44, ICAM-1, Transferrin receptor, LDL related protein, Leptin receptor, Insulin-like growth factor receptor, Diphtheria toxin receptor, Neonatal Fc Receptor or Nicotinic acetylcholine receptor.

In a preferred embodiment, the nanocarrier is characterized in that the nanocarrier is a nanoparticle selected from the group comprising: polymeric nanoparticles, lipid nanoparticles or inorganic nanoparticles.

The **third embodiment** of the invention refers to any of the protein-based biosensor or nanocarriers described above, for use in a method for quantifying or monitoring a substate or neurotransmitter *in vivo,* preferably in the brain, or in the blood-brain barrier.

In a preferred embodiment, the protein-based biosensor or nanocarrier is used in the diagnosis of neurological or neurodegenerative disorders.

One of the main applications of the proposed MRI biosensors is to measure glutamate transport across the BBB. Since endothelial cells at the BBB are the final hurdle for eliminating the excess glutamate from brain parenchyma to the blood stream, the capacity of BBB to transport glutamate into the bloodstream, or to retain glutamate at the brain parenchyma, could be an excellent target to identify patients in risk of suffering neurological diseases. The inventors consider several strategies to deliver the sensor to the BBB using nanoparticles; for instance, the use of nanoparticles targeted to the BBB to promote drug delivery to the brain. Since the glutamate sensors have been designed to measure glutamate at the BBB, instead of measuring the brain parenchyma, the developed nanoparticles are not requested to cross the BBB for the diagnosis, avoiding one of the main hurdles in brain delivery and also the interaction with cells of the nervous system. They designed a nanoparticle where the sensor is encapsulated inside the particle and surrounded by molecules with targeting activity for the BBB.

The nanoparticle composition plays a critical role in two important features for a successful brain imaging: i) protecting the sensor against degradation and rapid elimination from the bloodstream after i.v. injection, and ii) delivering the sensor to the BBB.

In that regard, the first point is achieved by encapsulating the sensor inside nanoparticles. The nanoparticles are usually composed by one of the following families, or a combination of them:
- Polymer: biocompatible and biodegradable polymers such as poly esters (PLA, PLGA. PLA-PLGA, PCL); polysaccharides; polyethylene glycol, polyglycerol, polystyrene
- Lipids and oils
- Inorganic nanoparticles (silicon, iron, gold, silver)

The second point regarding the active targeting to the brain is achieved by decorating the surface of the nanoparticle with a targeting molecule. The targeting moiety is an antibody, a peptide, an aptamer or a small molecule with affinity for one receptor at the BBB. The cell receptors used for brain targeting are the following: VCAM-1, E-Selectin, PECAM-1, CD44, ICAM-1, Transferrin receptor, LDL related protein, Leptin receptor, Insulin-like growth factor receptor, Diphtheria toxin receptor, Neonatal Fc Receptor, Nicotinic acetylcholine receptor.

The **fourth embodiment** of the invention refers to an *in vitro* method for determining the presence of a substate or neurotransmitter in a biological sample obtained from the subject, which comprises:
a. Bringing any of the protein-based biosensors described above in contact with the biological sample obtained from the subject suspected of comprising the substate or neurotransmitter, and
b. Wherein the identification of a shift in the MRI signal relative to the MRI signal obtained in the absence of the substrate or neurotransmitter, is an indication of the presence substate or neurotransmitter in the sample.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.

### Description of the figures

**Figure 1****.** Schematic representation of the synthesis of the glutamate MRI senor. The initial point is the Gltl protein domain present in the iGluSnFr protein. Upon binding with glutamate, the Gltl folds and causes an increase of the MRI signal provided by Gd-DOTA attached to the 27 lysine residues in the protein.
**Figure 2****.** Structure of the fluorescent glutamate sensor (iGluSnFr) and the synthesized MRI Sensor characterized by SEQ ID NO: 1. The yellow regions in the proteins are the histidine tag, the red regions are the glutamate recognition subunit (Gltl), the green regions are the GFP subunit. Aminoacid K* represent the lysins that have been modified with DOTA.
**Figure 3****.** Fluorescent signal provided by the sensor with and without 250 µM glutamate at different sensor concentrations.
**Figure 4****.** Fluorescent signal provided by the sensor (0.43 nM) at different concentrations of glutamate (glu) and arginine (arg).
**Figure 5****.** Comparison of the T1 relaxivity plots between bovine serum albumin (BSA) reacted with Gd in the presence and absence of DOTA. The highest slope was observed for BSA-DOTA-Gd.
**Figure 6****.** Comparison of the T1 relaxivity plots of the MRI sensor in presence (+glu) and absence (-glu) of 1 mM glutamate.
**Figure 7****.** T1 signal of the MRI glutamate sensor at increasing glutamate concentrations. The measurements were performed in triplicate. The standard deviation is represented in the error bars.
**Figure 8****.** Left: T1 contrast of the sensor, GOT and Albumin labelled with DOTA-Gd, expressed as the percentage variation in T1 measured without glutamate and with 250 µM glutamate divided by the signal without glutamate. Right: Representative images obtained by MRI for the sensor.

### Detailed description of the invention

The present invention is illustrated by means of the Examples set below without the intention of limiting its scope of protection.

### Example 1. Material and Methods

### Example 1.1. Materials

The plasmid in an agar stab of bacteria pRSET.GltI253-cpGFP.L1LV/L2NP was provided by Loren Looger (Addgene plasmid #41733; RRID:Addgene_41733). *E. coli* BL21(DE3)pLysS competent cells, PureYield Plasmid MiniPrep, enzymes EcoRI and Ndel, DNA Ladder and BlueJuice 10x were purchased from Promega. Luria-Bertani (LB) agar and SYBR were purchased from Invitrogen. LB culture medium, ampicillin, isopropyl-β-D-1-thiogalactopyranoside (IPTG), lysozyme (Lysozyme from chicken egg), imidazole, gadolinium (III) chloride and poly(lactic-co-glycolic acid) (PLGA) (Resomer) were purchased from Sigma-Aldrich. Protease inhibitor (cOmplete Tablets) and nickel resin (cOmplete His-Tag Purification Resin) were purchased from Roche. Ultraflitration tubes were purchased from Millipore, 1,4,7,10-Tetraazacyclododecane-1,4,7,10-tetraacetic acid mono-N-hydroxysuccinimide ester (DOTA-NHS) were purchased from CheMatech and dialysis bags (Float-A-Lyzer G2) were purchased from Spectra/Por.

### Example 1.2. Fluorescent sensor synthesis

The fluorescent sensor was synthesized and purified following standard protocols for protein synthesis, starting with the plasmid in *E. coli* bacterial culture. Plasmid containing iGluSnFR (Addgene, plasmid #41733) were transformed into *E. coli* BL21(DE3)pLysS competent cells, seed on LB agar with 100 µg/mL ampicillin and grown overnigth at 37 °C. The isolated colonies are selected and grown on LB medium with 100 µg/mL ampicillin overnight at 37 °C with shaking at 220 rpm. To select recombinant bacteria their plasmid was isolated using the commercial kit "PureYield Plasmid MiniPrep" and digested using EcoRI and Ndel. Agarose gel electrophoresis was performed with the enzyme digestion product, using SYBR as a contrast agent, a DNA Ladder as fragment length marker and BlueJuice 10x as loading buffer. Correctly transformed bacteria will have a banding pattern with sizes 1673 bp and 2759 bp.

Transformed bacteria were incubated overnight at 37°C in LB culture medium with 100 µg/mL ampicillin under agitation. This pre-culture was used as inoculum at a ratio of 1:50 in LB culture medium with 100 µg/mL, which was maintained at 37 °C with agitation at 220 rpm until an OD600 of between 0.4 and 0.6 was reached. Once the optical density was reached, sensor synthesis was induced by adding 1 mM IPTG and overnight cultured at 37 °C and shaking at 220 rpm. Finally, the cells were centrifuged, and the pellets were collected.

The pellets were resuspended in 10 mL of phosphate buffer with 10 mg lysozyme (Lysozyme from chicken egg) and protease inhibitor (cOmplete Tablets), and the mixture was sonicated in cycles of 0.5 s and 80% amplitude for 5 min. Then, the mixture was centrifuged at 15000 g, 4 °C for 30 min and the supernatant was recovered. The supernatant was passed through a 0.22 µm pore filter and purified using a nickel resin (cOmplete His-Tag Purification Resin). The glutamate fluorescent sensor was eluted using the same phosphate buffer with a 250 mM concentration of imidazole. The sensor was concentrated with 10 KDa filters at 15000 g and the medium was replaced with storage buffer (50 mM HEPES; 200 mM NaCl and pH 7.5). Finally, glycerol was added to a final volume of 10% and the purified fluorescent sensor was stored at -80°C.

### Example 1.3. MRI sensor synthesis

The fluorescent sensor (2mg, 0.03 µmol) was initially dissolved in 400 µL of carbonate buffer (50 mM, pH 9) and then 3 µmol (100 equivalents) of DOTA-NHS dissolved in 100 µL of DMSO were added. The reaction was carried out for 5 hours at 4 °C under stirring. Then, the mixture was transferred to 1 mL of acetate buffer (50 mM, pH 5) and 1.65 µmol of gadolinium (III) were added to the mixture and maintained overnight at 4 °C with constant mixing. Finally, unbounded gadolinium (III) and DOTA were removed, and the medium was changed to PBS by 10 KDa pore dialysis using Float-A-Lyzer G2.

GOT-DOTA-Gd was synthesized from GOT (0.5 mg, 0.03 µmol) was initially dissolved in 400 µL of carbonate buffer (50 mM, pH 9) and then 3 µmol (100 equivalents) of DOTA-NHS dissolved in 100 µL of DMSO were added. The reaction was carried out for 5 hours at 4 °C under stirring. Then, the mixture was transferred to 1 mL of acetate buffer (50 mM, pH 5) and 1.65 µmol of gadolinium (III) were added to the mixture and maintained overnight at 4 °C with constant mixing. Finally, unbounded gadolinium (III) and DOTA were removed, and the medium was changed to PBS by 10 KDa pore dialysis using Float-A-Lyzer G2.

BSA-DOTA-Gd was synthesized from BSA (2 mg, 0.03 µmol) was initially dissolved in 400 µL of carbonate buffer (50 mM, pH 9) and then 3 µmol (100 equivalents) of DOTA-NHS dissolved in 100 µL of DMSO were added. The reaction was carried out for 5 hours at 4 °C under stirring. Then, the mixture was transferred to 1 mL of acetate buffer (50 mM, pH 5) and 1.65 µmol of gadolinium (III) were added to the mixture and maintained overnight at 4 °C with constant mixing. Finally, unbounded gadolinium (III) and DOTA were removed, and the medium was changed to PBS by 10 KDa pore dialysis using Float-A-Lyzer G2.

### Example 1.4. MRI characterization

All studies were conducted on a Bruker Biospec 9.4 T MR scanner (horizontal bore magnet with 12 cm wide Bruker BioSpin) equipped with actively shielded gradients (440 mT m⁻¹). For the *in vitro* acquisition data, a radiofrequency resonator was used as transmitter-receiver (quadrature volume coil 7 cm in diameter).

The T1 relaxivity (r1) or T2 relaxivity (r2), as a measure of its value as MRI contrasts, were evaluated at 9.4 T in an agar (1.6 % w/v) mold with multiple wells following a procedure described elsewhere. Sensors were first diluted in water and placed in eppendorf tubes and then inserted in the mold. The mold was then sealed with liquid agar and cooled to room temperature. T2-weighted images (T2-wi) were acquired using the multi-slice multi-spin-echo (MSME) sequence with 11.32 ms of echo time (ET), 3 s of repetition time (RT),16 echoes with 11.32 ms echo spacing (ES), 50 kHz spectral bandwidth (SB), flip angle (FA) of 90°, 14 slices of 1 mm, 1 average. T2-wi were obtained with a field of view (FOV) of 7.5×7.5 cm² (with saturation bands to suppress signal outside this FOV), and a matrix size of 300×300, giving an in-plane resolution of 250 µm/pixel and implemented without fat suppression. T1-weighted images (T1-wi) were acquired using a rapid acquisition with relaxation enhancement-variable (RARE-VTR) sequence with an ET = 17.48 ms, RT =1 s, NA = 1, number of repetitions (NR)=1, SW = 50 kHz, rare factor (RF)=4; 6 T1 experiments, RT= 900, 2000, 3500, 5000, 7000, 11000 ms, and with the same T2 geometry.

### Example 2. Results

### Example 2.1. Fluorescent characterization of the existing iGluSnFR glutamate sensor

The inventors of the present invention worked with the hypothesis that attaching gadolinium (Gd) to a glutamate-binding protein would result in a new protein that, upon contact with glutamate would experience a conformational change that would shift the MRI signal provided by the Gd attached to the structure **(****Figure 1****).**

To test this hypothesis, the inventors generated an MRI glutamate sensor by labelling a glutamate transporter protein (Gltl) with Gd, using DOTA as a chelating agent **(****Figure 2****).** The fluorescent glutamate sensor iGluSnFR was used as a starting point, since apart from containing a Gltl domain, it also has a fluorescent protein reporter (GFP) that was useful for preliminary tests.

The fluorescent glutamate sensor iGluSnFR was synthesized and isolated through the histidine tag following standard procedures. The inventors tested the glutamate sensing capacity of the sensor by measuring the changes in fluorescence upon incubation with glutamate **(****Figure 3****).** They also tested the changes in fluorescence in the presence of other amino acids to evaluate the selective binding of the sensor to glutamate **(****Figure 4****).** As expected, fluorescent intensity increased in a sensor-dependent manner in the presence of glutamate **(****Figure 3****)** and in a glutamate-dependent manner **(****Figure 4****).**

### Example 2.2. Synthesis of an MRI glutamate sensor based on the existing iGluSnFR glutamate sensor

The new MRI glutamate sensor was synthesized by reacting the 52 lysine residues on the iGluSnFR sensor with the chelator DOTA, by stable amide bonds between lysine and DOTA. Next, Gd was attached to the sensor through the DOTA chelator **(****Figure 1** and **Figure 2****).**

The reaction of lysine with DOTA-NHS was tested first in a model protein, in this case albumin, to optimize the reaction conditions. Albumin was first reacted with DOTA-NHS and then labelled with Gd through the DOTA-Lysine residues. As a control, albumin without DOTA was also labelled with Gd. We measured the MRI T1 relaxivities and we observed a higher signal for DOTA labelled albumin, demonstrating the need of a chelating agent for labelling proteins with Gd **(****Figure 5****).** Following the same reaction as for BSA, we attached DOTA-Gd to the lysines of iGluSnFr to produce the MRI glutamate sensor.

### Example 2.3. In vitro evaluation of the sensing properties of the MRI glutamate sensor

To investigate the sensing properties of the MRI glutamate sensor, the inventors analysed the MRI signal produced by the sensor as a function of the concentration of the sensor. As shown in **Figure 6****,** the slope and the signal increased in the presence of glutamate, indicating that the MRI signal produced by the sensor in the presence of glutamate differed from that generated in the absence of glutamate.

The MRI sensing properties were also evaluated by incubating the sensor with different concentrations of glutamate **(****Figure 7****).** An increase in the concentration of glutamate was associated to a reduction in the MRI signal, which resulted from a glutamate-induced conformational change in Gltl.

The glutamate sensing capacity of the MRI glutamate sensor was then compared to that provided by other proteins labelled with DOTA-Gd: BSA-DOTA-Gd and GOT-DOTA-Gd. BSA-DOTA-Gd was used as a negative control, since BSA does not have affinity for glutamate. On the other hand, the glutamic oxalacetate transaminase (GOT) is an enzyme involved in glutamate metabolism. GOT-DOTA-Gd was used to evaluate whether the same synthetic strategy applied to another glutamate binding protein could result in a reliable glutamate sensor. The inventors compared the T1 signal of the MRI sensor in presence and absence of 250 uM glutamate with the signal provided by BSA-DOTA-Gd and GOT-DOTA-Gd in the same conditions. As expected, a very small variation in the MRI signal was detected upon incubation of the control sensor (BSA-DOTA-Gd) with glutamate, while a higher variation was detected for the MRI glutamate sensor (comprising the Gltl domain) and the GOT-DOTA-Gd sensor **(****Figure 7****).** Notably, the sensing profile of the MRI glutamate sensor (comprising the Gltl domain) was superior to that observed for the GOT-DOTA-Gd.

Together, the analysis described above indicate that i) a synthetic glutamate sensor containing a glutamate transporter (Gltl-DOTA-Gd), produces an intense glutamate-dependent MRI signal, ii) a synthetic glutamate sensor generated with a glutamate binding protein which is not a glutamate-specific transporter (GOT-DOTA-Gd), also generates a glutamate-induced MRI signal, although to a lower level than the one generated when using the glutamate transporter, and iii) a protein sensor generated with a protein that has no specificity for glutamate (BSA-DOTA-Gd), does not produce a glutamate-dependent MRI signal, indicating that a glutamate binding protein is required for such signal to occur.

## Claims

1. Protein-based biosensor comprising a substrate-binding protein bound to an MRI contrast agent by means of a chelating agent.

2. Protein-based biosensor, according to claim 1, comprising a neurotransmitter binding protein bound to an MRI contrast agent by means of a chelating agent.

3. Protein-based biosensor, according to any of the previous claims, comprising a glutamate binding protein bound to an MRI contrast agent by means of a chelating agent.

4. Protein-based biosensor, according to any of the previous claims, wherein the substrate-binding protein is bound to an MRI contrast agent by means of a cyclic chelating agent.

5. Protein-based biosensor, according to any of the previous claims, wherein the chelating agent is dodecane tetraacetic acid (DOTA).

6. Protein-based biosensor, according to any of the previous claims, wherein the MRI contrast agent is a gadolinium-based MRI contrast agent.

7. Protein-based biosensor, according to any of the previous claims, comprising a glutamate binding protein bound to gadoteric acid (DOTA-Gd) that consists of dodecane tetraacetic acid (DOTA) as a chelating agent and gadolinium.

8. Protein-based biosensor, according to any of the previous claims, **characterized in that** the dodecane tetraacetic acid (DOTA) is bound to the glutamate binding protein by means of amide bonds with lysine amino acids.

9. Protein-based biosensor, according to any of the previous claims, wherein the glutamate binding protein is **characterized by** SEQ ID NO: 1.

10. Nanocarrier comprising the protein-based sensor of any of the claims 1 to 9, **characterized in that** it is functionalized with a ligand capable to bind to the blood-brain barrier.

11. Nanocarrier, according to claim 10, **characterized in that** the ligand is selected from the list comprising: an antibody, a peptide, an aptamer or a small molecule with affinity for at least one receptor at the blood-brain barrier selected from the list comprising: VCAM-1, E-Selectin, PECAM-1, CD44, ICAM-1, Transferrin receptor, LDL related protein, Leptin receptor, Insulin-like growth factor receptor, Diphtheria toxin receptor, Neonatal Fc Receptor or Nicotinic acetylcholine receptor.

12. Nanocarrier, according to any of the claims 10 or 11, **characterized in that** the nanocarrier is a nanoparticle selected from the group comprising: polymeric nanoparticles, lipid nanoparticles or inorganic nanoparticles.

13. Protein-based biosensor of claims 1 to 9, or nanocarrier of claims 10 to 12, for use in a method for quantifying or monitoring a substate or neurotransmitter *in vivo,* preferably in the brain, or in the blood-brain barrier.

14. Protein-based biosensor of claims 1 to 9, or nanocarrier of claims 10 to 12, for use, according to claim 13, in the diagnosis of neurological or neurodegenerative disorders.

15. *In vitro* method for determining the presence of a substate or neurotransmitter in a biological sample obtained from the subject, which comprises:
a. Bringing the protein-based biosensor of claims 1 to 9 in contact with the biological sample obtained from the subject suspected of comprising the substate or neurotransmitter, and
b. Wherein the identification of a shift in the MRI signal relative to the MRI signal obtained in the absence of the substrate or neurotransmitter, is an indication of the presence substate or neurotransmitter in the sample.
